# EUROPÄISCHE PATENTSCHRIFT

(11) **EP 0 283 754 B1**
(45) Veröffentlichungstag und Bekanntmachung des Hinweises auf die Patenterteilung: **02.02.1994**
(21) Anmeldenummer: 88102770.0
(22) Anmeldetag: 25.02.1988
(51) Int. Cl.: C07D 487/22, C09B 47/00, C07F 11/00, C07B 41/00, B01J 31/22, C07D 301/03

(54) **Wolfram-oxo-porphyrin-Komplexe**
Tungsten oxo-porphyrin complexes
Complexes de wolfram oxo-porphyrine

(30) Priorität: 25.03.1987 DE 3709831
(43) Veröffentlichungstag der Anmeldung: 28.09.1988
(73) Patentinhaber: Degussa Aktiengesellschaft, 60311 Frankfurt (DE)
(72) Erfinder: Buchler, Johan, Prof. Dr., D-6100 Darmstadt (DE); Herget, Gerhard, D-6100 Darmstadt (DE); Schmidt, Manfred, Dr., D-6460 Gelnhausen (DE); Prescher, Günter, Dr., D-6450 Hanau 9 (DE)

(56) Entgegenhaltungen:
- EP-A- 0 283 753
- DE-A- 3 800 974
- US-A- 4 614 723
- JOURNAL OF CHEMICAL SOCIETY, CHEMICAL COMMUNICATIONS, 1985, Seiten 888-889
- RUSSIAN JOURNAL OF INORGANIC CHEMISTRY, Band 30, 1985, Seiten 352-353; T.N. LOMOVA et al.: "A spectro-photometric study of complexes of molybdenum and tungsten with tetraphenylporphine in proton-donating solvents"
- INORGANIC CHEMISTRY, Band 18, 1979, Seiten 2156-2157; E.B. FLEISCHER et al.: "Synthesis and oxidative demetalation of two new tungsten porphyrins"

## Beschreibung

Die Erfindung betrifft neue Übergangsmetall-Komplexverbindungen mit
- 5,10,15,20-Tetraphenylporphyrinen oder
- 5,10,15,20-Tetra(4-pyridyl)-porphyrinen als Liganden,
welche gegebenenfalls am Zentralatom ein Anion tragen und welche sich als Katalysator zur Epoxidation von Olefinen mit Wasserstoffperoxid eignen.

Olefine (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US-PS 2 412 136 sowie DE-AS 11 39 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit in alkalischem Medium und nachfolgender Behandlung mit Basen herstellen.

Ein weiterer Prozeß beruht auf der Umsetzung von Olefinen mit organischen Hydroperoxiden in Gegenwart eines Katalysators (siehe DE-AS 14 68 012).

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE-PS 535 068).

Damit verbundene Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. In diesem Zusammenhang wird beispielsweise hingewiesen auf GB-PS 837 464, bei welchem die in J.A.C.S., Band 59, Seiten 2342 bis 2344 (1937) beschriebenen verschiedenen Metallkatalysatoren verwendet werden, auf US-PS 2 786 854, wonach Wolframsäure eingesetzt wird, auf US-PS 2 833 787, wonach saure Salze von Metallen aus der Gruppe VI des Periodensystems der Elemente, z. B. von Wolfram und Molybdän, angewandt werden, auf BE-PS 860 776, wonach Wolfram- und Molybdän-haltige Verbindungen verwendet werden, auf US-PS 3 993 673, wonach Arsenhaltige Katalysatoren verwendet werden, auf US-PS 3 953 362, wonach ein Molybdän-haltiger Katalysator angewandt wird, auf US-PS 4 026 908, wonach Quecksilberderivate plus eine Molybdän-, Wolfram-, Vanadin- oder Titanverbindung angewandt wird, auf US-PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US-PS 3 778 451, wonach Molybdän-, Wolfram-, Vanadin-, Niob-, Tantal , Uran- und Rheniumverbindungen eingesetzt werden.

Diese Stoffe sind zwar katalytisch aktiv, doch haben aus verschiedenen Gründen die damit grundsätzlich ausführbaren Verfahren keinen Eingang in die Technik gefunden. In Verbindung mit Wasserstoffperoxidlösungen wird durch sie entweder das Wasserstoffperoxid rasch zersetzt oder nur eine unbefriedigende Epoxidationsgeschwindigkeit erreicht. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metallporphyrinkomplexen als Katalysatoren durchzuführen. Als zur Umsetzung mit Epoxidationsmitteln wie Jodosobenzol, Alkalimetallhypochlorit sowie organische Hydroperoxide geeignete Metallkatalysatoren sind z. B. das Chloro-Eisen (III)-tetraphenylporphyrin (FeCl)(TPP), das Chloro-Mangan (III)-tetraphenylporphyrin (MnCl)(TPP) oder das ChloroChrom(III)-tetraphenylporphyrin (CrCl)(TPP) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli, J.F.; Mansuy, D., J. Chem. Soc., Chem. Commun. 1985, 888). Allerdings wirken diese Katalysatoren stark zersetzend auf H₂O₂, so daß die bezüglich Wasserstoffperoxid erreichbaren Selektivitäten nur sehr gering sind, es sei denn, es werden aufwendig substituierte Porphyrinliganden verwendet.

Auch Oxo-Metallporphyrinkomplexe, wie Oxo-chloro(5,10,15, 20-tetraphenylporphyrinato)-molybdän(V) (O=Mo(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Methoxo-oxo(5,10,15,20-tetraphenylporphyrinato-molybdän(V) zur Epoxidierung des olefins Cyclohexen zu verwenden, schlug jedoch fehl: Es konnte keine Epoxidation beobachtet werden (F.Varescon, These, L'Université Claude Bernard-Lyon I, 1982).

Gegenstand der Erfindung sind Oxowolfram(V)-Porphyrin-Komplexe WO(P)X mit (P)²⁻ = Dianion des
- 5,10,15,20-Tetraphenylporphyrins oder des
- 5,10,15,20-Tetra(4-pyridyl)-porphyrins als Liganden,
welche am Zentralatom Wolfram ein Anton X⁻ aus der Reihe X = F, Cl, Br, J, C₂H₅O, C₃H₇O, t-C₄H₉O, AcO, SCN, CN, und 0/2⁻ (µ-oxo) tragen.

Ein weiterer Gegenstand sind Oxowolfram(V)-Porphyrin-Komplexe WO(P)X mit (P)²⁻ = Dianion von
- 5,10,15,20-Tetraphenylporphyrinen oder
- 5,10,15,20-Tetra(4-pyridyl)-porphyrinen als Liganden,
welche am Zentralatom Wolfram ein Anion X⁻ tragen, in denen jeweils Wasserstoffatome oder freie Elektronenpaare an den Phenyl- bzw. Pyridylgruppen der Liganden ein- oder mehrmals substituiert sind durch Halogen, Hydroxy, Carboxy, Cyano, Rhodano, Nitro, C₁-C₆-Alkyl, Trihalogenmethyl, C₁-C₆-Alkoxy, C₁-C₆-Alkansulfonyloxy, Aminocarbonyl, einen oder zwei C₁-C₆-Alkylreste enthaltendes Aminocarbonyl, C₁-C₆-Alkylcarbonyl, Amino, Di-C₁-C₆-Alkylamino, (C₁-C₆-Alkyl)₃N, C₁-C₆-Alkanoylamino, C₁-C₆-Alkyl-C₁-C₆-alkanoylamino, C₁-C₆-Alkansulfonylamino, C₁-C₆-Alkyl-C₁-C₆-alkansulfonylamino, Aminosulfonyl, einen oder zwei C₁-C₆-Alkylreste enthaltendes Aminosulfonyl,
C₁-C₆-Alkoxysulfonyl (-SO₂-O-C₁-C₆-Alkyl), Sulfo oder C₁-C₆-Alkansulfonyl und zwei dieser Reste auch die Methylendioxygruppe sein können und X jeweils OH, CH₃O, C₆H₅O, F, Cl, Br, J, C₂H₅O, C₃H₇O, t-C₄H₉O, AcO, SCN, CN, und 0/2⁻(µ-oxo) ist.

Durch die sterischen und elektronischen Effekte der genannten und anderer Substituenten am Phenyl- bzw. Pyridylrest des 5,10,15,20-Tetraphenylporphyrins bzw. 5,10,15,20-Tetra(4-pyridyl)-porphyrins kann man, angepaßt an das jeweilige Olefin, die katalytischen Eigenschaften steuern und optimieren.

Die erfindungsgemäßen Katalysatoren sind neue Stoffe. Davon ist eine Reihe nach bekannten Literaturmethoden in großer Reinheit zugänglich:
J.W. Buchler et al., Chem. Ber., 1973, 106, 2710;
Liebigs Ann. Chem. 1971, 745, 135;
Inorg. Nucl. Chem. Lett., 1972, 8, 1073;
K. Rohbock, Dissertation, RTWH Aachen, 1972.
Die verschiedenen Porphyrinliganden werden, sofern sie nicht käuflich sind, nach
Adler et al., J. Org. Chem. 32, 476 (1967) bzw.
Adler et al., J. Heterocyl. Chem. 5, 669 (1968) dargestellt und sofern erforderlich, von Chlorin (Porphyrin mit einem teilhydrierten Pyrrolglied) befreit (K.M. Smith et al., Tetrahedron Lett., 30, 2887 (1973)).

T.N. Lomova, N.I. Volkova, B.D. Berezin, Russ. J. Inorg. Chem. 30, 352 - 354 (1985) und E.B. Fleischer, R.D. Chapman, M. Krishnamurthy, Inorg. Chem. 18, 2156 - 2159 (1979) haben Wolfram-oxo-Komplexe mit den gemäß Erfindung verwendeten Liganden, jedoch mit den Gegenionen OH⁻, CH₃O⁻ und C₆H₅O⁻ beschrieben.

Im wesentlichen verwenden die obengenannten Autoren als Wolframquelle Wolframhexacarbonyl W(CO)₆ bzw. Kaliumenneachlorodiwolframat(III) K₃W₂Cl₉ oder Wolframhexachlorid WCl₆. Während K₃W₂Cl₉ und WCl₆ nur mit relativ großem Arbeitsaufwand hergestellt werden können, gelingt die Umsetzung zwischen W(CO)₆ und dem jeweiligen Porphyrinliganden nur durch sehr langes Rückflußkochen in Dimethylformamid. Es bestand daher ein erhebliches Bedürfnis nach einfacheren und billigeren Herstellungsmethoden.

Ein weiterer wichtiger Gegenstand der Erfindung ist daher ein Verfahren zur Herstellung von Wolfram-oxo-Komplexen allgemein, insbesondere aber der erfindungsgemäßen neuen Wolfram-Komplexe. Das Verfahren ist dadurch gekennzeichnet, daß man ein Mol 5,10,15,20-Tetraphenylporphyrin oder 5,10,15,20-Tetra(4-pyridyl)porphyrin mit mindestens einem Mol Wolframpentahalogenid in einem hochsiedenden, alle Reaktanden lösenden Solvens, vorzugsweise Benzonitril oder Trichlorbenzol, bei Temperaturen von 160 - 250° C, vorzugsweise 180 - 220° C, umsetzt, das Solvens vorzugsweise im Vakuum entfernt, den Rückstand nach Aufnehmen in einem organischen Lösungsmittel durch Säulenchromatographie fraktioniert, dann die in der Hauptfraktion vorliegenden, hinsichtlich der Zusammensetzung des Anions X⁻ noch inhomogenen Komplexe WO(P)X entweder jeweils mit einer äquimolaren Menge einer gegebenenfalls in situ erzeugten Alkalimetallverbindung, welche das gewünschte Anion X⁻ in Bindung mit dem Alkalimetall enthält, bei 0 - 100° C, vorzugsweise 40 - 60° C, in einem Lösungsmittelgemisch, das sowohl WO(P)X als auch die Alkalimetallverbindung löst, umsetzt und daraus die das gewünschte Anton X⁻ tragenden Oxowolfram(V)-Porphyrin-Komplexe WO(P)X durch Einengen zur Kristallisation bringt oder aber jeweils mit einem Überschuß der zum gewünschten Anion X⁻ korrespondierenden Brönsted-Säure HX bei 20 - 180° C, vorzugsweise 40 - 120° C, in Lösungsmittelgemischen behandelt, die sowohl den Halogeno-oxowolfram-Komplex WO(P)X (X = F, Cl, Br, J) als auch die Brönstedsäure HX lösen, und daraus die das gewünschte Anion X⁻ tragenden Komplexe WO(P)X durch Einengen zur Kristallisation bringt.

Ein weiterer neuer Herstellungsweg für Wolfram-oxo-Komplexe allgemein, insbesondere aber für solche gemäß der Erfindung besteht darin, daß man ein Mol des jeweiligen Porphyrinliganden zu einer siedenden Lösung von mindestens einem Mol Wolframtetrachlorid in Benzonitril oder Trichlorbenzol gibt, wobei das Wolframtetrachlorid zuvor in situ durch Umsetzung von 1 Mol Wolframhexacarbonyl und 2 Mol Wolframhexachlorid in diesem Lösungsmittel hergestellt wird und nicht isoliert werden muß, und diese Lösung bei Temperaturen von 160 - 250, vorzugsweise 180 - 220° C, umsetzt, das Solvens vorzugsweise im Vakuum entfernt, den Rückstand nach Aufnehmen in einem organischen Lösungsmittel durch Säulenchromatographie fraktioniert, die in der Hauptfraktion vorliegenden, hinsichtlich der Zusammensetzung des Anions X⁻ noch inhomogenen Komplexe 40(P)X entweder jeweils mit einer äquimolaren Menge einer gegebenenfalls in situ erzeugten Alkalimetallverbindung, welche das gewünschte Anion X⁻ in Bindung mit dem Alkalimetall enthält bei 0 - 100, vorzugsweise 40 - 60° C, in einem Lösungsmittelgemisch, das sowohl WO(P)X als auch die Alkalimetallverbindung löst, umsetzt und daraus die das gewünschte Anion X⁻ tragenden Komplexe WO(P)X durch Einengen zur Kristallisation bringt oder aber jeweils mit einem Überschuß der zum gewünschten Anion X⁻ korrespondierenden Brönsted-Säure HX bei 20 - 180° C, vorzugsweise 40 - 120° C, in Lösungsmittelgemischen behandelt, die sowohl den Halogeno-oxowolfram(V)-Komplex als auch die Brönsted-Säure lösen, und daraus die das gewünschte Anion X⁻ tragenden Komplexe WO(P)X durch Einengen zur Kristallisation bringt.

Wie erwähnt, kann die zur Homogenisierung der Zusammensetzung des Anions verwendete Alkalimetallverbindung auch in situ erzeugt werden. Damit ist z. B. die Bildung von Methoxiden aus "methanolischem Kali" gemeint, d. h. die in Beispiel 4 (Darstellung von WO(TTP)OMe) verwendete Mischung aus Dichlormethan/Methanol und wäßrigem Kaliumhydroxid, die Methoxid-Ionen enthält.

Während des Wolfram-Einbaus dringen Sauerstoff und Wasser in ausreichender Menge aus der Luft bzw. aus den Chemikalien oder Gefäßmaterialien in die Reagenzien ein, so daß im Zuge der Reaktion Wolfram(IV) zu Wolfram(V) autoxidiert wird und Dihalogenowolfram-Gruppen zu Oxowolfram-Gruppen hydrolysiert werden. Es besteht jedoch auch die Möglichkeit, den Reaktanden im Verfahren gemäß Anspruch 3 direkt Wasser und im Verfahren gemäß Anspruch 5 Sauerstoff und Wasser zuzugeben.

Die Reaktionszeiten für den Einbau des Wolframs in das jeweilige Ausgangsporphyrin nach den Methoden des Anspruchs 3 oder 5 liegen etwa im Bereich von 3 bis 20 h, meist aber im Bereich von 6 - 12 h.

Der Endpunkt der Reaktion kann spektralphotometrisch festgestellt werden. Es erscheint das UV/Vis-Spektrum des gebildeten Wolfram-Porphyrins anstelle des UV/Vis-Spektrums des Ausgangsporphyrins. Einen groben Anhaltspunkt liefert bereits die dunkelgrüne Farbe der Lösung des Endprodukts.

Wesentlich für die Umsetzung von Wolframhexahalogenid mit Wolframhexacarbonyl ist die Verwendung von koordinierenden Lösungsmitteln, wie organische Nitrile, z. B. Benzonitril, höhersiedende Äther und Thioäther z. B. Diethylenglycoldimethylether oder Pentamethylensulfid, Dimethylformamid etc. Günstig sind Lösungsmittel mit einem etwa oberhalb 180° C liegenden Siedepunkt.

Als zur Vereinheitlichung der Zusammensetzung des Anions geeignete Brönsted-Säuren kommen alle Verbindungen in Frage, welche die in Anspruch 2 genannten Anionen bzw. aber auch OH⁻, OCH₃⁻ und C₆H₅O⁻ (bei Substitution der Grundkörper gemäß Anspruch 2) in Kombination mit dem Proton enthalten.

Bei der nach beiden erfindungsgemäßen Herstellungsvarianten durchgeführten Säulenchromatographie werden übliche Adsorbentien, wie Aluminiumoxide oder Kieselgele, eingesetzt. Als Eluens für die Abtrennung des nicht umgesetzten Porphyrins wird ein schwach polares Lösungsmittel, wie Dichlormethan oder Toluol und für die Abtrennung der Wolframkomplexfraktion (Hauptfraktion) ein Gemisch aus einem schwach polaren (z. B. Chloroform oder Dichlormethan) und einem stark polaren Lösungsmittel (Methanol, Ethanol, Propanol oder Aceton) eingesetzt. Besonders gut eignet sich ein Gemisch aus 95 Vol.% Chloroform und 5 Vol.% Methanol.

Mit den beschriebenen neuen Katalysatoren können die verschiedensten Olefine mit Wasserstoffperoxid epoxidiert werden. Dabei kann in organischen Lösungsmitteln, insbesondere solchen, welche ein Übertreten von Wasserstoffperoxid aus wäßriger Phase in die organische Phase gestatten, gearbeitet werden.

Die anzuwendenden Mengen an Katalysator können in einem weiten Bereich liegen. Die im Einzelfall anzuwendende Katalysatorkonzentration kann, entsprechend dem Typ der vorgesehenen Wolfram-porphyrinverbindung sowie entsprechend der Reaktivität des jeweils umzusetzenden Olefins gewählt werden. Sie liegt in einem Konzentrationsbereich, der im allgemeinen 1/10000 bis 1/2 mol, vorzugsweise 1/5000 bis 1/5 mol pro mol Wasserstoffperoxid beträgt.

Die einmal eingesetzten Katalysatoren lassen sich nach geeigneter Abtrennung des Reaktionsgemisches für weitere Ansätze wiederverwenden.

Die ohnehin hohe Selektivität läßt sich durch Zusatz von geringen Mengen, vorzugsweise von 0,1 bis 10 mol eines heterocyclischen Amins, bezogen auf 1 mol des Katalysators, insbesondere von äquimolaren Mengen einer Verbindung aus der Familie des Pyridins, wie 2,6-Dimethylpyridin, 2,6-Ditertiärbutylpyridin, 3,5-Dimethylpyridin sowie die drei Picoline, die 4-Halogenopyridine sowie die Salze des 2,2-Bipyridyls,oder des Imidazols, z. B. das Imidazol selbst, verbessern. Die Konzentration des Olefins im Reaktionssystem ist nicht kritisch, das molare Verhältnis zwischen Olefin und Wasserstoffperoxid kann 1 : 30 bis 30 : 1 betragen.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150, vorzugsweise 20 bis 120, insbesondere 20 bis 80° C. Die Reaktionszeiten liegen normalerweise bei 10 Minuten bis 24 Stunden. Die Reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die mit der Erfindung erzielbaren Vorteile sind:
- Sehr kurze Reaktionszeiten
- Hohe Selektivität (kaum Nebenprodukt)
- Niedrige Katalysatorkonzentration
- Hohe chemische Stabilität des Katalysators, insbesondere gegenüber dem Epoxidationsmittel
- Keine oder nur minimale H₂O₂-Zersetzung
- Leichte Abtrennbarkeit und Wiederverwendbarkeit des Katalysators
- Vereinfachte Synthese der Katalysatoren
Die Erfindung wird im folgenden anhand von Ausführungsbeispielen zu den einzelnen Herstellungsmethoden gemäß Erfindung unter Charakterisierung der Verfahrensprodukte weiter erläutert.

Die folgenden Ausführungsbeispiele geben komplette Darstellungsvorschriften für bestimmte Wolfram-oxo-Komplexe, und darin jeweils die Kombination der Wolfram-Einführung und der Einführung eines definierten Anions in den Komplex, an.

Zur Charakterisierung der Verbindungen dient hauptsächlich die UV/Vis-Spektroskopie. Die Lage der Banden im sichtbaren Spektralbereich wird bei W^{v}-Porphyrinen sehr stark vom Anion beeinflußt, das in trans-Stellung zur Oxogruppe steht.

### Beispiel 1

### Herstellung von Bromo-oxo-5,10,15,20-tetra(p-tolyl)porphyrinatowolfram(V), WO(TTP)Br

a) Einführung des Wolframs:
Zu einer unter Stickstoff bereiteten Lösung von 1.17 g (2.0 mmol) Wolframpentabromid (WBr₅) in 250 ml wasserfreiem Benzonitril (getrocknet über CaH₂) gab man 200 mg Tetra(p-tolyl)porphyrin, H₂(TTP), und erhitzte das Gemisch zum Sieden unter Rückfluß. In einer unter dem Rückflußkühler angebrachten Extraktionshülse befanden sich weitere 470 mg H₂(TTP), die allmählich in das Reaktionsgefäß gespült wurden (eingesetzte Gesamtmenge H₂(TTP): 1 mmol). Nach 6 h Erhitzen zum Sieden ließ man abkühlen, destillierte im Hochvakuum das Benzonitril ab, nahm den trockenen Rückstand in Chloroform auf und chromatographierte an Al₂O₃ (Stufe III, neutral, Säule 3.8 x 12 cm) und erhielt folgende Fraktionen:
1. Rotviolett, H₂(TTP), 10 mg, Eluens: Chloroform ;
2. Dünkelgrün, Wolfram(V)-Porphyrin, Eluens: Dichlormethan/Methanol;

b) Vereinheitlichung des Axialliganden Bromid:
Fraktion 2 wurde im Vakuum vom Lösungsmittel befreit und der Rückstand aus Dichlormethan/Methanol unter Zusatz von 4 ml 40 % wäßriger Bromwasserstoffsäure kristallisiert. Man erhielt 909 mg (97%) WO(TTP)Br in Form violettgrün schimmernder Kristalle.

| C₄₈H₃₆BrN₄OW (948.59) | | | | |
|---|---|---|---|---|
| Ber. | C 60.78; | H 3.82; | Br 8.42; | N 5.91 % |
| Gef. | C 60.79; | H 3.55; | Br 8.71; | N 6.21 % |

UV/Vis (Tolüöl): λ max (log ε ) = 654 (3.99), 608 (3.96), 476 (5.20), 316 nm (4.53). IR (CsI: 961 cm⁻¹ (W=O). Massenspektrum (Feldionen-Desorption): A = 868 (¹⁸⁴WO(TTP)⁺; Molekül-Ion nicht beobachtbar.

### Beispiel 2

### Herstellung von Chloro-oxo-5,10,15,20-tetra(p-tolyl)porphyrinatowolfram(V), WO(TTP)Cl

a) Einführung des Wolframs :
   Entsprechend Beispiel 1, Teil a) wurden 1.17 g (2.0 mmol) WBr₅ mit 670 mg H₂(TTP) (1 mmol) zur Umsetzung gebracht, aufgearbeitet und chromatographiert. Die rotviolette Fraktion 1 enthielt 8 mg H₂(TTP).
b) Vereinheitlichung des Axialliganden Chlorid:
   Die grüne Fraktion 2 lieferte nach dem Entfernen des Lösungsmittels im Vakuum und Umkristallisieren des Rückstandes aus Dichlormethan/Methanol unter Zusatz von 5 ml 37% wäßriger Salzsäure 823 mg (91 %) WO(TTP)Cl.
   UV/Vis (Toluol) λ max (log ε ) = 646 (3.92), 602 (3.93), 560 (3.61), 468 (5.33), 344 (4.42), 312 nm (4.47). Massenspektrum (Feldionen-Desorption): A = 903 (¹⁸⁴WO(TTP)³⁵Cl⁺), 868 (¹⁸⁴WO(TTP)⁺).

### Beispiel 3

### Herstellung von Acetato-oxo-5,10,15,20-tetra(p-tolyl)porphyrinatowolfram(V), WO(TTP)OAc

a) Einführung des Wolframs:
   Zur tiefblauen Lösung von 529 mg (1.33 mmol) WCl₆ in 250 ml wasserfreiem Benzonitril (getrocknet über CaH₂) gab man unter Argon und unter Rühren 235 mg (0.67 mmol) W(CO)₆. In wenigen Minuten schlug die Farbe der Lösung nach hellgelb um.* Nach Zugabe von 261 mg (0.39 mmol) H₂(TTP) erhitzte man 14 h unter Rückfluß, entfernte das Lösungsmittel im Hochvakuum, nahm den Rückstand in Chloroform auf und chromatographierte an Aluminiumoxid (Stufe III, neutral 2.5 x 20 cm) und erhielt folgende Fraktionen:
   1. violettrot, H₂(TTP), 5 mg, mit Dichlormethan als Eluens;
   2. grün, vermutlich ein Gemisch von WO(TTP)Cl und WO(TTP)OMe, mit Dichlormethan/Methanol (95:5) als Eluens.

b) Einführung des Acetations:
   Fraktion 2 wurde im Vakuum vom Lösungsmittel befreit und der Rückstand aus Dichlormethan/Eisessig kristallisiert. Man erhielt 304 mg (84 %) WO(TTP)OAc.
   UV/Vis (Toluol unter Zusatz von 1 Tropfen Eisessig direkt in die 1mm-Küvette), λ max (log ε ) = 632 (4.05), 588 (4.51), 456 (5.49), 304 nm (4.58). Massenspektrum (Feldionen-Desorption): A = 927 (¹⁸⁴WO(TTP)OAc⁺; Molekül-Ion), 868 (¹⁸⁴WO(TTP)⁺). IR (CsI): 1650 (C=O, OAc), 961 cm⁻¹ (W=O).

*) In Analogie zur Darstellung von WBr₄, aus WCl₆ und W(CO)₆ in Chlorbenzol
F.A. Cotton, S. Koch, K. Mertis, M. Millar, G. Wilkinson, J. Am. Chem. Soc. 99, 4989 - 4992 (1977) und WCl₄(PrCN)₂ aus Propionitril (PrCN) und WCl₅ (E.A. Allen, B.J. Brisdon, G.W.A. Fowles, J. Chem. Soc. 1964, 4531 - 4534),
oder WCl₄(MeCN)₂ aus WCl₅ und W(CO)₆ in Acetonitril (MeCN)
(M.A.S. King, R.E. McCarley, Inorg. Chem. 12, 1972 (1973)) ist anzunehmen, daß sich an dieser Stelle WCl₄(PhCN)₂ gebildet hat.

### Beispiel 4

### Herstellung von Methoxo-oxo-5,10,15,20-tetra(p-tolyl)porphyrinatowolfram(V), WO(TTP)OMe

a) Einführung des Wolframs:
   Entsprechend Beispiel 3 wurden 530 mg (1.33 mmol) WCl₆, 235 mg (0.67 mmol) W(CO)₆ und 261 mg H₂(TTP) (0.39 mmol) in 13 h zur Umsetzung gebracht und aufgearbeitet. Die Chromatographie an Aluminiumoxid (Stufe III, neutral, 3.5 x 20 cm) lieferte 2 Fraktionen:
   1. violettrot, H₂(TTP), 5 mg, mit Dichlormethan als Eluens;
   2. grün, vermutlich WO(TTP)Cl, mit Dichlormethan/Methanol (95:5) als Eluens. Fraktion 2 wurde im Vakuum vom Lösungsmittel befreit. Der rohe Rückstand wog 349 mg (98 %, bezogen auf WO(TTP)Cl).
b) Vereinheitlichung des Axialliganden Methoxid:
   Umkristallisieren des Rückstands aus Dichlormethan/Methanol unter Zusatz von 1 ml 10 % wäßriger Kaliumhydroxid-Lösung ergab violettgrüne Kristalle von WO(TTP)OMe.
   UV/Vis (Toluol/Methanol, 98:2), λ max (log ε ): = 624 (3.78), 582 (3.88), 446 (5.30), 308 (4.40). IR (KBr): 2770 (C-H von MeO), 904 (W=O), 1100 (C-O von MeO), 446 (W-O von WOMe).

### Beispiel 5

### Herstellung von Rhodano-oxo-5,10,15,20-tetraphenylporphyrinatowolfram(V), WO(TPP)SCN.

a) Einführung des Wolframs:
   Entsprechend Beispiel 1, Teil a) wurden 2,34 g (4.0 mmol) WBr₅ mit 1,34 g (2.0 mmol) H₂(TPP) zur Umsetzung gebracht, aufgearbeitet und chromatographiert. Die rotviolette Fraktion 1 enthielt H₂(TPP).
b) Einführung des Axialliganden Rhodanid:
   Die grüne Fraktion 2 wurde im Vakuum vom Lösungsmittel befreit und der Rückstand in Dichlormethan/Methanol (1 : 1) gelöst, mit einem Überschuß gesättigter methanolischer KSCN-Lösung versetzt und 6 h unter Rückfluß erhitzt. Anschließend wurde abfiltriert, mit Wasser gewaschen und im Hochvakuum getrocknet. Man erhielt WO(TPP)SCN in 77 %iger Ausbeute.

UV/VIS (CH₂Cl₂) λ max (log ε ) = 668 (3.83), 643 (3.83), 595 (3.79), 464 (5.0), 310 nm (4.48).
Massenspektrum (Elektronenstoß-Ionisation, Verdampfung von einem Rheniumdraht): A = 812 (¹⁸⁴WO(TPP)⁺; Molekül-Ion nicht beobachtbar, (negatives Fast-Atom-Bombardement-Spektrum, FAB): A = 58(SCN⁻). IR(KBr): 2027(CN), 961 cm⁻¹ (W = O).

Die folgenden Beispiele 6 - 8 betreffen die Anwendung des neuen Herstellungsverfahrens zur Gewinnung von Octaethylporphyrin-Komplexen. Die Beispiele 9 - 22 beziehen sich auf erfindungsgemäße neue Katalysatorstoffe und deren neuartige Herstellung.

Zur Charakterisierung der neuen Wolfram-Porphyrine wird zusätzlich noch folgendes bemerkt:

Die µ-Oxo-Komplexe sind durch Überführung in die entsprechenden Einkern-Komplexe und durch die UV/VIS- und IR-Spektren hinreichend charakterisiert. Bei einigen TPP-bzw. TTP-Komplexen liegen die C- und N-Werte der Elementaranalysen etwas zu hoch. Dies ist auf einen geringfügigen Benzonitril-Gehalt des Ausgangs-µ-Oxo-Komplexes zurückzuführen. Generell darf man bei den schwerverbrennbaren Porphyrin-Komplexen nicht die Genauigkeit der Elementaranalysen erwarten, die man von rein organischen Verbindungen gewohnt ist.

Die massenspektrometrischen Daten beziehen sich auf das häufigste Wolfram-Isotop ¹⁸⁴W; bei Chlorgehalt auf ³⁵Cl. µ-Oxo-Komplexe lassen sich wegen zu geringer Flüchtigkeit massenspektrometrisch nicht charakterisieren. Wegen der leichten Abspaltbarkeit bestimmter Axial-Liganden sind bei einkernigen Wolframporphyrinen oft nur die Peaks der entsprechenden Oxowolframporphyrin-Kationen beobachtbar. Die UV/VIS-Spektren wurden in Dichlormethan gemessen.

### Beispiel 6

### µ-Oxobis [oxo(2,3,7,8,12,13,17,18-octaethylporphyrinato)-wolfram(V)] , [WO(OEP)] ₂O

In einem 100 ml-Zweihalskolben mit Rückflußkühler und Stickstoffeinleitungsrohr wurden 641 mg (1.2 mmol) H₂(OEP), 1.3 g (2.2 mmol) WBr₅ in 75 ml 1.2.4-Trichlorbenzol unter Rühren zum Sieden erhitzt. Fortlaufend entnommene Proben wurden UV/VIS-spektroskopisch untersucht. Die Spektren nach 3 h bzw. 4.5 h Reaktionszeit unterschieden sich nicht und ließen nur noch geringe Spuren freien Porphyrins erkennen. Die Reaktion wurde abgebrochen und das Lösungsmittel im Hochvakuum entfernt. Der dunkelbraune Rückstand wurde in CH₂Cl₂ aufgenommen und filtriert. Man chromatographierte an Al₂O₃ (Aktivität III-n, 3.8 x 13 cm): 1. Fraktion, grünlichhellblau, nach Verlassen der Säule allmählich nach rot umschlagend, H₂(OEP), 10 mg, Eluens CH₂Cl₂; 2. Fraktion, grün, Eluens CH₂Cl₂/MeOH 95 : 5, MeOH-Anteil allmählich auf 8 % gesteigert. Die 2. Fraktion wurde zur Trockne eingedampft, in 40 ml CH₂Cl₂ aufgenommen und mit einer Lösung von 2 g KOH in 20 ml Wasser 18 h gerührt. Man entfernte das Lösungsmittel durch gelindes Erwärmen, filtrierte den ausgefallenen Komplex ab und wusch mit destilliertem Wasser neutral. Kristallisation aus CH₂Cl₂/Toluol lieferte 764 mg (86 %) µ-oxo-Komplex [WO(OEP)] ₂O als rot-violettes feinkristallines Pulver.
C₇₂H₈₈N₈O₃W₂ (1481.2)
UV/VIS ( λ max, log ε ): 366 (4.74), 430 (5.09), 558 (4.11), 660 (3.32) nm.
IR(KBr): 646, 725 cm⁻¹ (typisch für µ-oxo-Systeme)

### Beispiel 7

### Acetatooxo(2,3,7,8,12,13,17,18-octaethylporphyrinato)-wolfram(V), WO(OEP)OAc

Eine Lösung von 222 mg (0.15 mmol) [WO(OEP)] ₂O in 30 ml CHCl₃ wurde mit 2 ml Eisessig und 50 mg Natriumacetat über Nacht gerührt. Dann entfernte man das Lösungsmittel und Teile der Essigsäure durch leichtes Erwärmen. Waschen mit destilliertem Wasser befreite den Komplex von anhaftender Essigsäure und Natriumacetat. Nach Trocknung im Vakuum und Kristallisation aus Toluol resultierten 202 mg (85 %) WO(OEP)OAc als violett schimmernde, plättchenförmige Kristalle.

| C₃₈H₄₇N₄O₃W (791.67) | | | |
|---|---|---|---|
| Ber.: | C 57.65 | H 5.98 | N 7.08 |
| Gef.: | C 56.95 | H 5.63 | N 7.15 |

UV/VIS ( λ max, log ε ): 324 (4.45), 442 (5.01), 565 (4.00), 600 (3.77) nm.
IR (KBr): 1660 (asymm. COO), 935 (W = O) cm⁻¹
MS (FD): 791 (¹⁸⁴WO(OEP)OAc⁺).

### Beispiel 8

### Perchloratooxo(2,3,7,8,12,13,17,18-octaethylporphyrinato)-wolfram(V), WO(OEP)OClO₃

Eine Lösung von 222 mg (0.15 mmol) [WO(OEP)] ₂O in 30 ml CHCl₃ wurde mit 3 ml Perchlorsäure (7 % wäßrige Lösung) über Nacht gerührt. Das Lösungsmittel wurde unter gelindem Erwärmen entfernt und der Rückstand mit destilliertem Wasser neutral gewaschen. Kristallisation aus Toluol lieferte 215 mg (86 %) WO(OEP)OClO₃ als violette, metallisch glänzende Plättchen.

| C₃₆H₄₄N₄O₅WCl (832.1) | | | |
|---|---|---|---|
| Ber.: | C 51.97 | H 5.33 | N 6.73 |
| Gefn.: | C 51.32 | H 5.47 | N 7.07 |

UV/VIS ( λ max, log ε): 328(4.52), 438(4.94), 562(4.00), 598(3.83) nm.
IR (KBr): ca. 1100 cm⁻¹ (W=O Bande liegt unter Porphyrinbanden)
MS (FD): 831 (¹⁸⁴WO(OEP)OClO₃⁺), 732 (¹⁸⁴WO(OEP)⁺).

### Beispiel 9

### µ-Oxobis [oxo(5,10,15,20-tetraphenylporphyrinato)-wolfram(V)] , [WO(TPP)] ₂O

In einem 500 ml-Dreihalskolben mit Rückflußkühler, Gaseinleitung und Stopfen (zur Probeentnahme) wurden 1.58 g (4 mmol) WCl₆, 0.7 g (2 mmol) W(CO)₆, 0.92 g H₂(TPP) (1.5 mmol) sowie 350 ml vorgetrocknetes Benzonitril unter Rühren und Durchleiten eines gelinden Stickstoffstromes zum Sieden erhitzt; UV/VIS-spektroskopisch ließ sich nach 11 h kein freies Porphyrin mehr nachweisen. Dann wurde das Lösungsmittel im Vakuum abgezogen und Lösungsmittelreste im Hochvakuum entfernt. Der bräunlich-grüne Rückstand wurde in CH₂Cl₂ aufgenommen und an Al₂O₃ (Aktivität III-n, 3.8 x 15 cm) chromatographiert: 1. Fraktion, rot, H₂(TPP), 11 mg Eluens CH₂Cl₂; 2. Fraktion, dunkelgrün, Eluens CH₂Cl₂/MeOH 95:5. Die Hauptfraktion wurde zur Trockne eingedampft, in 50 ml CH₂Cl₂ aufgenommen und mit einer Lösung von 4 g KOH in 30 ml Wasser 18 h gerührt. Nach Abdampfen des Lösungsmittels filtrierte man den angefallenen Komplex ab und wusch mit destilliertem Wasser neutral. Kristallisation aus CH₂Cl₂/Toluol lieferte 1080 mg (88 %) µ-oxo-Komplex als blau-grünes feinschuppiges Pulver.
C₈₈H₅₆N₈O₃W₂ (1641.1)
UV/VIS ( λ max, log ε ): 314(4.70), 444(5.39), 582(4.08), 620(3.96) nm.
IR (KBr): 670, 725 cm⁻¹ (spezifisch für µ-oxo-Komplexe)

### Beispiel 10

### Chlorooxo(5,10,15,20-tetraphenylporphyrinato)-wolfram(V), WO(TPP)Cl

Eine Lösung von 246 mg (0.15 mmol) [WO(TPP)] ₂O in 30 ml CHCl₃ wurde mit 4 ml 37 % wäßriger Salzsäure über Nacht gerührt. Nach Verdampfen des Lösungsmittels wurde der Rückstand mit destilliertem Wasser neutral gewaschen. Kristallisation aus Toluol lieferte 229 mg (90 %) WO(TPP)Cl als dunkelgrüne metallisch schimmernde Kristalle.

| C₄₄H₂₈N₄OWCl (832.1) | | | |
|---|---|---|---|
| Ber.: | C 62.32 | H 3.33 | N 6.61 |
| Gef.: | C 64.5 | H 3.41 | N 6.84 |

UV/VIS ( λ max, log ε ): 316(4.57), 466(5.23), 600(3.95), 632(3.90) nm.
IR (KBr): 950 cm⁻¹ (W = O)
MS (FD): 847 (¹⁸⁴WO(TPP)Cl⁺), 812 (¹⁸⁴WO(TPP)⁺).

### Beispiel 11

### Fluorooxo(5,10,15,20-tetraphenylporphyrinato)wolfram(V), WO(TPP)F

Eine Lösung von 197 mg (0.12 mmol) [WO(TPP)] ₂O in 15 ml CHCl₃ wurde in ein Teflongefäß gegeben und 2 ml Flußsäure (40 %) zugefügt. Der Ansatz wurde über Nacht gerührt.

Das Lösungsmittel und ein Großteil der Flußsäure wurde durch Erwärmen im Wasserbad abgedampft. Der Rückstand wurde mit destilliertem Wasser neutral gewaschen und nach Trocknung aus Toluol kristallisiert. Man erhielt 184 mg (92 %) WO(TPP)F als blau-violette Schuppen.
C₄₄H₂₈N₄OWF (831.6)
UV/VIS ( λ max, log ε ): 328(4.44), 446(5.40), 580(4.01), 622(3.83) nm
IR (KBr): 950 cm⁻¹ (W = O)
MS (FD): 831 (¹⁸⁴WO(TPP)F⁺).

### Beispiel 12

### µ-Oxobis [oxo{5,10,15,20-tetra(p-tolyl)porphyrinato}-wolfram(V) , [WO(TPP)] ₂O

In einem 500 ml-Dreihalskolben mit Rückflußkühler, Gaseinleitungsrohr und Stopfen (zur Probeentnahme) wurden 1.98 g (5 mmol) WCl₆, 0.88 g (2.5 mmol) W(CO)₆, 1.34 g (2 mmol) H₂(TTP) sowie 400 ml getrocknetes Benzonitril unter Durchleiten eines gelinden Stickstoffstromes und Rühren zum Sieden erhitzt. Der Reaktionsverlauf wurde UV/VIS-spektroskopisch verfolgt; nach 12 h ließ sich kein freies Porphyrin mehr nachweisen. Die Reaktion wurde abgebrochen, das Lösungsmittel im Vakuum entfernt und letzte Lösungsmittelreste im Hochvakuum abgezogen. Der bräunlich-grüne Rückstand wurde in CH₂Cl₂ aufgenommen und an Al₂O₃ (Aktivität III-n, 3.8 x 15 cm) chromatographiert:
1. Fraktion, hellrot, H₂(TTP), 9 mg, Eluens CH₂Cl₂;
2. Fraktion, dunkelgrün, Eluens CH₂Cl₂/MeOH 95:5. Die Hauptfraktion wurde zur Trockne eingedampft, in ca. 80 ml CH₂Cl₂ aufgenommen und anschließend mit einer Lösung von 4 g KOH in 30 ml Wasser über Nacht gerührt.

Man entfernte das organische Lösungsmittel unter leichtem Erwärmen, filtrierte den ausgefallenen Komplex ab (Glasfritte Porengröße 4) und wusch mit destilliertem Wasser neutral. Kristallisation aus CH₂Cl₂/Toluol lieferte 1595 mg (91 %) [WO(TTP)] ₂O als blau-grünes feinkristallines Pulver.
C₉₆H₇₂N₈O₃W₂ (1753.4)
UV/VIS ( λ max, log ε ): 308(4.81), 448(5.28), 586(4.12), 6.26(4.09) nm
IR (KBr): 670, 725 cm⁻¹ (spezifisch für µ-oxo-Systeme)

### Beispiel 13

### Chlorooxo [5,10,15,20-tetra(p-tolyl)porphyrinato] wolfram(V), WO(TTP)Cl

Eine Lösung von 263 mg (0.15 mmol) [WO(TTP)] ₂O in 30 ml CHCl₃ wurde mit 4 ml 37 % wäßriger Salzsäure über Nacht gerührt. Nach Abdampfen des Lösungsmittels wurde der Rückstand mit destilliertem Wasser neutral gewaschen. Kristallisation aus Toluol ergab 255 mg (94 %) WO(TTP)Cl als dunkelgrüne schuppige Kristalle.

| C₄₈H₃₆N₄OWCl (904.14) | | | |
|---|---|---|---|
| Ber.: | C 63.74 % | H 4.01 % | N 6.20 % |
| Gef.: | C 66.79 % | H 3.99 % | N 6.56 % |

UV/VIS ( λ max, log ε ): 314(4.60), 470(5.26), 604(3.95), 650(4.00) nm
IR: 950 cm⁻¹, (W = O)
MS: 904, (¹⁸⁴WO(TTP)Cl)

### Beispiel 14

### Perchloratooxo [5,10,15,20-tetra(p-tolyl)porphyrinato]-wolfram(V), WO(TTP)OClO₃

Eine Lösung von 264 mg (0.15 mmol) [WO(TTP)] ₂O in 30 ml CHCl₃ wurde mit 4 ml wäßriger 7 % Perchlorsäure 18 h gerührt. Man dampfte das Lösungsmittel unter leichtem Erwärmen ab und wusch den Rückstand mit destilliertem Wasser neutral. Kristallisation aus Toluol lieferte 247 mg (85 %) WO(TTP)ClO₄ als tief-violette metallisch glänzende Plättchen.

| C₄₈H₃₆N₄O₅WCl (968.1) | | | |
|---|---|---|---|
| Ber.: | C 59.95 | H 3.75 | N 5.79 |
| Gef.: | C 61.78 | H 3.84 | N 6.40 |

UV/VIS ( λ max, log ε ): 308(4.49), 458(4.92), 632(3.72), 650(4.00) nm
IR (KBr): ca. 1100 cm⁻¹ (Bande für koordiniertes Perchlorat liegt unter Porphyrinbanden)
MS (FD): 868 (¹⁸⁴WO(TTP)⁺)

### Beispiel 15

### Acetatooxo [5,10,15,20-tetra(p-tolyl)porphyrinato]-wolfram (V), WO(TTP)OAc

Eine Lösung von 263 mg (0.15 mmol) [WO(TTP)] ₂O in 30 ml CHCl₃ wurde mit 2 ml Eisessig sowie 50 mg Natriumacetat versetzt und über Nacht gerührt. Man entfernte das Lösungsmittel und Teile der Essigsäure durch gelindes Erwärmen. Der Rückstand wurde durch Waschen mit destilliertem Wasser vom Natriumacetat und anhaftender Essigsäure befreit, im Vakuum getrocknet und aus Toluol kristallisiert. Man erhielt 248 mg (99 %) WO(TTP)OAc als feinkristallines dunkelgrünes Kristallpulver.

| C₅₀H₃₉N₄O₃W | | | |
|---|---|---|---|
| Ber.: | C 64.73 | H 4.24 | N 6.04 |
| Gef.: | C 67.17 | H 4.29 | N 6.48 |

UV/VIS ( λ max, log ε ): 306(4.49), 456(5.27), 590(3.96), 632(3.90) nm
IR (KBr): 1660 (asymm. COO), 950 (W = O) cm⁻¹
MS (FD): 868 (¹⁸⁴WO(TTP)⁺)

### Beispiel 16

### µ-Oxobis [oxo{5,10,15,20-tetra(p-methoxyphenyl)porphyrinato}wolfram(V) , [WO(TAP)] ₂O

Eine Lösung von 258 mg (0.5 mmol) WBr₅ in 30 ml Benzonitril wurde 1.5 h unter Rückfluß erhitzt und nach Zugabe von 170 mg (0.25 mmol) H₂(TAP) weitere 3.5 h am Sieden gehalten. Nach Abdestillieren des Lösungsmittels im Hochvakuum wurde der Rückstand in CHCl₃ aufgenommen und an Al₂O₃ (Aktivität II, basisch, 2.6 x 4.5 cm) chromatographiert.
1. Fraktion: rotviolett H₂(TAP) (Eluens: CH₂Cl₂)
2. Franktion: grün WO(TAP)X (Eluens: CH₂Cl₂/MeOH 99/1)
3. Fraktion: grün WO(TAP)X (Eluens: CH₂Cl₂/MeOH 95/5)
Da die Fraktionen 2 und 3 im UV/VIS-Spektrum dieselben Banden zeigten, wurden sie vereinigt und das Lösungsmittel im Vakuum abdestilliert. Der Rückstand wurde in CHCl₃ gelöst, mit 20 ml verd. wäßrigem KOH versetzt und 2 h gerührt. Das CHCl₃ wurde durch gelindes Erwärmen entfernt, der ausgefallene Niederschlag abfiltriert und mit destilliertem Wasser neutral gewaschen. Man erhielt 70 mg (15 %) grüne Schüppchen von [WO(TAP)] ₂O.
UV/VIS ( λ max, log ε ): 318(4.76), 450(5.54), 470(5.07), 588(4.15), 632(4.20), 672(3.99)nm
IR (KBr): 650, 719 cm⁻¹ (typisch für µ-oxo Systeme)

### Beispiel 17

### Perchloratooxo [5,10,15,20-tetra(p-methoxyphenyl)porphyrinato] wolfram(V), WO(TAP)OClO₃

Eine Lösung von 66 mg (0.035 mmol) [WO(TAP)] ₂O in 10 ml CHCl₃ wurde mit 2 ml wäßrigem 7 % HClO₄ versetzt. Unter ständigem Rühren erwärmte man leicht, bis das CHCl₃ verdunstet war. Der ausgefallene Komplex wurde abfiltriert, mit destilliertem Wasser neutral gewaschen und aus CHCl₃ kristallisiert. Man erhielt 57 mg (80 %) WO(TAP)OClO₃ als grüne Schüppchen.
C₄₈H₃₆N₄O₉WCl (1032.1)
UV/VIS ( λ max, log ε ): 308(4.57), 462(5.24), 590(4.02), 636(4.04) nm
IR (KBr): ca. 1100 cm⁻¹ (Perchlorat- und W=O Banden liegen unter Porphyrinbanden)
MS (FD): 1031 (¹⁸⁴WO(TAP)OClO₃⁺), 932 (¹⁸⁴WO(TAP)⁺)

### Beispiel 18

### µ-Oxobis [oxo{5,10,15,20-tetra(p-chlorphenyl)porphyrinato}wolfram(V) , [WO(Tp-ClPP)] ₂O

538 mg (1 mmol) WBr₅ und 378 mg (0.5 mmol) H₂(Tp-ClPP) wurden in 70 ml TCB gelöst und 1 h unter Rückfluß erhitzt. Die Reaktion wurde UV/VIS-spektroskopisch kontrolliert. Das TCB wurde im Hochvakuum abdestilliert und der Rückstand in CHCl₃ aufgenommen.

Chromatographiert wurde an Al₂O₃ (Aktivität-III, neutral, 3.6 x 12 cm):
1. Fraktion: braun, nicht näher identifizierbar (Eluens: CHCl₃)
2. Fraktion: grün, WO(Tp-ClPP)X (Eluens: CHCl₃).
Das CHCl₃ wurde im Vakuum entfernt. Der Rückstand wurde in CHCl₃ und 20 ml verdünnter KOH aufgenommen und die Lösung eingedunstet. Der verbliebene Rückstand wurde mit destilliertem Wasser aufgeschlämmt, abfiltriert, neutral gewaschen und aus Benzol/Cyclohexan kristallisiert. Man erhielt 412 mg (86 %) [WO(Tp-ClPP)] ₂O als schuppiges Pulver.

| C₈₈H₆₀N₈Cl₈O₃W₂ (1928.8) | | | |
|---|---|---|---|
| Ber.: | C 55.08 | H 2.5 | N 5.84 |
| Gef.: | C 51.03 | H 2.27 | N 5.33 (Substanz enthielt noch Aluminiumoxid) |

UV/VIS ( λ max, log ε ): 326(4.72), 446(5.61), 466(5.11), 578(4.29), 618(4.10) nm
IR (KBr): 723, 652 cm⁻¹ (typisch für µ-oxo Systeme)

### Beispiel 19

### Methoxo-oxo [5,10,15,20-tetra(p-chlorphenyl)porphyrinato]-wolfram(V), WO(Tp-ClPP)OMe

316 mg (0.164 mmol) [WO(Tp-ClPP)] ₂O wurden in 30 ml CHCl₃ gelöst und mit 20 ml MeOH zum Sieden erhitzt. Danach wurde das Lösungsmittel im Vakuum entfernt und der Rückstand aus MeOH umkristallisiert. Man erhielt 240 mg (75 %) grüne Plättchen des Methoxokomplexes.

| C₄₅H₂₇N₄Cl₄O₂W (981.4) | | | |
|---|---|---|---|
| Ber.: | C 55.08 | H 2.75 | N 5.71 |
| Gef.: | C 52.46 | H 2.36 | N 5.46 |
| (Substanz enthielt noch Aluminiumoxid, da aus µ-oxo-Komplex dargestellt). | | | |

UV/VIS ( λ max, log ε ): 324(4.52), 444(4.79), 466(5.35), 578(4.04), 620(3.83) nm
IR (KBr): 2795 (OMe), 920 (W = O) cm⁻¹
MS (FD): 979 (¹⁸⁴WO(Tp-ClPP)OMe⁺)

### Beispiel 20

### Perchlorato-oxo [5,10,15,20-tetra(p-chlorphenyl)porphyrinato] wolfram(V), WO(Tp-ClPP)OClO₃

Eine Lösung von 232 mg (0.12 mmol) [WO(Tp-ClPP)] ₂O in 15 ml CHCl₃ wurde mit 2 ml 7 % wäßriger HClO₄ versetzt. Unter ständigem Rühren erwärmte man leicht, bis das CHCl₃ verdunstet war. Der ausgefallene Komplex wurde abfiltriert, mit destilliertem Wasser neutral gewaschen und aus CHCl₃ kristallisiert. Man erhielt 225 mg (88 %) WO(Tp-ClPP)OClO₃ in Form von schwarzen Kristallen.

| C₄₄H₂₄N₄Cl₅O₅W (1049.8) | | | | | |
|---|---|---|---|---|---|
| Ber.: | C 50.35 | H 2.28 | N 5.33; | C:H = 22.08; | C:N = 9.44 |
| Gef.: | C 45.77 | H 1.99 | N 4.74; | C:H = 23.0; | C:N = 9.65 |
| Substanz enthielt noch Aluminiumoxid. | | | | | |

UV/VIS ( λ max, log ε ): 314(4.50), 456(5.26), 586(4.07), 628(3.78) nm
IR (KBr): ca. 1100 cm⁻¹ (Perchlorat- und W=O Banden liegen unter Porphyrinbanden)

### Beispiel 21

### Acetatooxo [5,10,15,20-tetra(p-chlorphenyl)porphyrinato]-wolfram(V), WO(Tp-ClPP)OAc

Eine Lösung von 293 mg (0.152 mmol) [WO(Tp-ClPP)] ₂O in 30 ml CHCl₃ wurde mit 2 ml Eisessig und 0.1 g NaOAc versetzt. Es wurde unter Erwärmen gerührt, bis das CHCl₃ verdunstet war. Der Rückstand wurde abfiltriert, mit destilliertem Wasser gewaschen und aus CHCl₃ kristallisiert, wobei man 239 mg (68%) WO(Tp-ClPP)OAc als grüne Täfelchen erhielt.

| C₄₆H₂₇N₄Cl₄O₃W (1009.4) | | | |
|---|---|---|---|
| Ber.: | C 54.75 | H 2.67 | N 5.55 |
| Gef.: | C 53.46 | H 2.57 | N 5.34 |

UV/VIS ( λ max, log ε ): 320(4.49), 454(5.39), 578(4.11), 620(3.83) nm
IR (KBr): 1660 (asymm. COO), 955 (W = O) cm⁻¹
MS (FD): 1007 (¹⁸⁴WO(Tp-ClPP)OAc⁺), 948 (¹⁸⁴WO(Tp-ClPP)⁺)

### Beispiel 22

### Bromooxo [5,10,15,20-tetra(p-chlorphenyl)porphyrinato]wolfram(V), WO(Tp-ClPP)Br

Eine Lösung von 259 mg (0.134 mmol) [WO(Tp-ClPP)] ₂O in 50 ml CHCl₃ wurde mit 5 ml 40 % wäßriger HBr unter Erwärmen gerührt, bis das CHCl₃ verdunstet war. Der Rückstand wurde abfiltriert, mit destilliertem Wasser neutral gewaschen und aus CHCl₃ kristallisiert. Man erhielt 244 mg (88 %) WO(Tp-ClPP)Br als schwarze Nädelchen.

| C₄₄H₂₄N₄Cl₄OWBr (1030.3) | | | |
|---|---|---|---|
| Ber.: | C 51.28 | H 2.33 | N 5.44 |
| Gef.: | C 47.93 | H 2.62 | N 4.88 |

UV/VIS ( λ max, log ε ): 324(4.51), 474(5.20), 606(3.95), 650(3.90) nm
IR (KBr): 958 cm⁻¹ (W = O)
MS (FD): 1027 (¹⁸⁴WO(Tp-ClPP)Br⁺), 948 (¹⁸⁴WO(Tp-ClPP)⁺).

## Patentansprüche

1. Oxowolfram(V)-Porphyrin-Komplexe WO(P)X mit (P)²⁻ = Dianion des
- 5,10,15,20-Tetraphenylporphyrins oder des
- 5,10,15,20-Tetra(4-pyridyl)-porphyrins als Liganden
welche am Zentralatom Wolfram ein Anion X⁻ aus der Reihe X = F, Cl, Br, J, C₂H₅O, C₃H₇O, t-C₄H₉O, AcO SCN, CN, und 0/2⁻ (µ-oxo) tragen.

2. Oxowolfram(V)-Porphyrin-Komplexe WO(P)X mit (P)²⁻ = Dianion von
- 5,10,15,20-Tetraphenylporphyrinen oder
- 5,10,15,20-Tetra(4-pyridyl)-porphyrinen als Liganden,
welche am Zentralatom Wolfram ein Anion X⁻ tragen, in denen jeweils Wasserstoffatome oder freie Elektronenpaare an den Phenyl- bzw. Pyridylgruppen der Liganden ein-oder mehrmals substituiert sind durch Halogen, Hydroxy, Carboxy, Cyano, Rhodano, Nitro, C₁-C₆-Alkyl, Trihalogenmethyl, C₁-C₆-Alkoxy, C₁-C₆-Alkansulfonyloxy, Aminocarbonyl, einen oder zwei C₁-C₆-Alkylreste enthaltendes Aminocarbonyl, C₁-C₆-Alkylcarbonyl, Amino, Di-C₁-C₆-Alkylamino, (C₁-C₆-Alkyl)₃N, C₁-C₆-Alkanoylamino, C₁-C₆-Alkyl-C₁-C₆-alkanoylamino, C₁-C₆-Alkansulfonylamino, C₁-C₆-Alkyl-C₁-C₆-alkansulfonylamino, Aminosulfonyl, einen oder zwei C₁-C₆-Alkylreste enthaltendes Aminosulfonyl, C₁-C₆-Alkoxysulfonyl (-SO₂-O-C₁-C₆-Alkyl), Sulfo oder C₁-C₆-Alkansulfonyl und zwei dieser Reste auch die Methylendioxygruppe sein können und X jeweils OH, CH₃O, C₆H₅O, F, Cl, Br, J, C₂H₅O, C₃H₇O, t-C₄H₉O, AcO, SCN, CN, und 0/2⁻(µ-oxo) ist.

3. Verfahren zur Herstellung der Oxowolfram(V)-Porphyrin-Komplexe WO(P)X, gemäß Anspruch 1 oder 2,
**dadurch gekennzeichnet,**
daß man ein Mol 5,10,15,20-Tetraphenylporphyrin oder 5,10,15,20-Tetra(4-pyridyl)-porphyrin mit mindestens einem Mol Wolframpentahalogenid in einem hochsiedenden, alle Reaktanden lösenden Solvens bei Temperaturen von 160 - 250° C umsetzt, das Solvens entfernt, den Rückstand nach Aufnehmen in einem organischen Lösungsmittel durch Säulenchromatographie fraktioniert, dann die in der Hauptfraktion vorliegenden, hinsichtlich der Zusammensetzung des Anions X⁻ noch inhomogenen Komplexe WO(P)X entweder jeweils mit einer äquimolaren Menge einer gegebenenfalls in situ erzeugten Alkalimetallverbindung, welche das gewünschte Anion X⁻ in Bindung mit dem Alkalimetall enthält, bei 0 - 100° C in einem Lösungsmittelgemisch, das sowohl WO(P)X als auch die Alkalimetallverbindung löst, umsetzt und daraus die das gewünschte Anion X⁻ tragenden Oxowolfram(V)-Porphyrin-Komplexe WO(P)X durch Einengen zur Kristallisation bringt oder aber jeweils mit einem Überschuß der zum gewünschten Anion X⁻ korrespondierenden Brönsted-Säure HX bei 20 - 180° C in Lösungsmittelgemischen behandelt, die sowohl den Halogeno-oxowolfram-Komplex WO(P)X (X = F, Cl, Br, J) als auch die Brönstedsäure HX lösen, und daraus die das gewünschte Anion X⁻ tragenden Komplexe WO(P)X durch Einengen zur Kristallisation bringt.

4. Verfahren nach Anspruch 3,
**dadurch gekennzeichnet,**
daß das alle Reaktanden lösende Solvens Benzonitril oder Trichlorbenzol ist, daß die Reaktanden in diesem Solvens bei Temperaturen von 180 bis 220° C umgesetzt werden und daß das Solvens im Vakuum entfernt wird.

5. Verfahren zur Herstellung der Oxowolfram(V)-Porphyrin-Komplexen WO(P)X gemäß Ansprüchen 1 oder 2,
**dadurch gekennzeichnet,**
daß man ein Mol des jeweiligen Porphyrinliganden zu einer siedenden Lösung von mindestens einem Mol Wolframtetrachlorid im Benzonitril oder Trichlorbenzol gibt, wobei das Wolframtetrachlorid zuvor in situ durch Umsetzung von 1 Mol Wolframhexacarbonyl und 2 Mol Wolframhexachlorid in diesem Lösungsmittel hergestellt wird und nicht isoliert werden muß, und diese Lösung bei Temperaturen von 160 - 250° C umsetzt, das Solvens im Vakuum entfernt, den Rückstand nach Aufnehmen in einem organischen Lösungsmittel durch Säulenchromatographie fraktioniert, die in der Hauptfraktion vorliegenden, hinsichtlich der Zusammensetzung des Anions X⁻ noch inhomogenen Komplexe WO(P)X entweder jeweils mit einer äquimolaren Menge einer gegebenenfalls in situ erzeugten Alkalimetallverbindung, welche das gewünschte Anion X⁻ in Bindung mit dem Alkalimetall enthält bei 0 - 100° C in einem Lösungsmittelgemisch, das sowohl WO(P)X als auch die Alkalimetallverbindung löst, umsetzt und daraus die das gewünschte Anion X⁻ tragenden Komplexe WO(P)X durch Einengen zur Kristallisation bringt oder aber jeweils mit einem Überschuß der zum gewünschten Anton X⁻ korrespondierenden Brönsted-Säure HX bei 20 - 180° C in Lösungsmittelgemischen behandelt, die sowohl den Halogeno-oxowolfram(V)-Komplex als auch die Brönstedsäure lösen, und daraus die das gewünschte Anion X⁻ tragenden Komplexe WO(P)X durch Einengen zur Kristallisation bringt.

6. Verfahren nach Anspruch 5,
**dadurch gekennzeichnet,**
daß man den jeweiligen Porphyrinliganden mit Wolframtetrachlorid in dem Lösungsmittel Benzonitril oder Trichlorbenzol bei Temperaturen von 180 bis 220° C umsetzt und das Lösungsmittel im Vakuum entfernt.

## Claims

1. Oxotungsten(V)-porphyrin complexes WO(P)X, where (P)²⁻ = dianion of
- 5,10,15,20-tetraphenylporphyrin or
- 5,10,15,20-tetra-(4-pyridyl)-phenylporphyrin as ligands,
which bear an anion X⁻ from the series X = F, Cl, Br, I, C₂H₅O, C₃H₇O, t-C₄H₉O, AcO, SCN, CN and/or 0/2⁻ (µ-oxo) at the central tungsten atom.

2. Oxotungsten(V)-porphyrin complexes WO(P)X, where (P)²⁻ = dianion of
- 5,10,15,20-tetraphenylporphyrins or
- 5,10,15,20-tetra-(4-pyridyl)-phenylporphyrins as ligands,
which bear an anion X⁻ at the central tungsten atom, in which hydrogen atoms or free electron pairs are substituted one or more times at the phenyl or pyridyl groups of the ligands by halogen, hydroxy, carboxy, cyano, thiocyano nitro, C₁₋₆ alkyl, trihalomethyl, C₁₋₆ alkoxy, C₁₋₆ alkanesulfonyloxy, aminocarbonyl, aminocarbonyl bearing one or two C₁₋₆ alkyl radicals, C₁₋₆ alkyl carbonyl, amino, di-C₁₋₆-alkylamino, (C₁₋₆ alkyl)₃N, C₁₋₆ alkanoylamino, C₁₋₆ alkyl-C₁₋₆ alkanoylamino, C₁₋₆ alkanesulfonylamino, C₁₋₆ alkyl-C₁₋₆ alkanesulfonylamino, aminosulfonyl, aminosulfonyl bearing one or two C₁₋₆ alkyl radicals, C₁₋₆ alkoxysulfonyl (-SO₂-O-C₁₋₆ alkyl), sulfo or C₁₋₆ alkanesulfonyl and two of these radicals may even be the methylenedioxy group and X is OH, CH₃O, C₆H₅O, F, Cl, Br, I, C₂H₅O, C₃H₇O, t-C₄H₉O, AcO, SCN, CN and 0/2⁻ (µ-oxo).

3. A process for the production of the oxotungsten(V)-porphyrin complexes WO(P)X claimed in claim 1 or 2, characterized in that one mol 5,10,15,20-tetraphenylporphyrin or 5,10,15,20-tetra-(4-pyridyl)- porphyrin is reacted with at least one mol tungsten pentahalide in a high-boiling solvent, which dissolves all the reactants, at temperatures of 160 to 250°C, the solvent is removed, the residue is dissolved in an organic solvent and fractionated by column chromatography, the complexes WO(P)X present in the main fraction, which are still inhomogeneous in regard to the composition of the anion X⁻, are either reacted with an equimolar quantity of an alkali metal compound optionally produced in situ, which contains the desired anion X⁻ bound to the alkali metal, at 0 to 100°C in a solvent mixture which dissolves both WO(P)X and the alkali metal compound and the oxotungsten(V)-porphyrin complexes WO(P)X bearing the desired anion X⁻ are crystallized therefrom by concentration or are treated with an excess of the Brönsted acid HX corresponding to the desired anion X⁻ at 20 to 180°C in solvent mixtures which dissolve both the halo-oxotungsten complex WO(P)X (X = F, Cl, Br, I) and the Brönsted acid HX and the complexes WO(P)X bearing the desired anion X⁻ are crystallized therefrom by concentration.

4. A process as claimed in claim 3, characterized in that the solvent dissolving all the reactants is benzonitrile or trichlorobenzene, in that the reactants are dissolved in this solvent at temperatures of 180 to 220°C and in that the solvent is removed in vacuo.

5. A process for the production of the oxotungsten(V)-porphyrin complexes WO(P)X claimed in claim 1 or 2, characterized in that one mol of the particular porphyrin ligand is added to a boiling solution of at least one mol tungsten tetrachloride in benzonitrile or trichlorobenzene, the tungsten tetrachloride being prepared beforehand by reaction of 1 mol tungsten hexacarbonyl and 2 mol tungsten hexachloride in this solvent without having to be isolated, and the resulting solution is reacted at temperatures of 160 to 250°C, the solvent is removed in vacuo, the residue is dissolved in an organic solvent and fractionated by column chromatography, the complexes WO(P)X present in the main fraction, which are still inhomogeneous in regard to the composition of the anion X⁻, are either reacted with an equimolar quantity of an alkali metal compound optionally produced in situ, which contains the desired anion X⁻ bound to the alkali metal, at 0 to 100°C in a solvent mixture which dissolves both WO(P)X and the alkali metal compound and the complexes WO(P)X bearing the desired anion X⁻ are crystallized therefrom by concentration or are treated with an excess of the Brönsted acid HX corresponding to the desired anion X⁻ at 20 to 180°C in solvent mixtures which dissolve both the halo-oxotungsten(V) complex and the Brönsted acid HX and the complexes WO(P)X bearing the desired anion X⁻ are crystallized therefrom by concentration.

6. A process as claimed in claim 5, characterized in that the particular porphyrin ligands are reacted with tungsten tetrachloride in benzonitrile or trichlorobenzene as solvent at temperatures of 180 to 220°C and the solvent is removed in vacuo.

## Revendications

1. Complexes oxo du tungstène (V) porphyrine WO(P)X avec (P)² = au dianion de
- la 5,10,15,20-tétraphénylporphyrine ou de
- la 5,10,15,20-tétra(4-pyridyl)porphyrine
comme ligands
qui portent sur l'atome central le tungstène, un anion X⁻ choisi dans la serie formée de F,Cl,Br,I,C₂H₅O, C₃H₇O, t-C₄H₉O, AcO, SCN, CN et 0/2- (µ-oxo).

2. Complexes oxo du tungstène (V) porphyrine WO(P)X avec (P)²⁻ est un dianion des
- 5,10,15,20-tétraphénylporphyrines ou des
- 5,10,15,20-tétra(4-pyridyl)porphyrines
qui portent sur l'atome central le tungstène un anion X⁻ dans lesquels respectivement les atomes d'hydrogène correspondant ou les paires d'électrons libres sur les groupes phényle ou pyridyle des ligands sont substitués une ou plusieurs fois par un halogène, un hydroxy, un carboxy, un cyano, un thiocyanato, un nitro, un alcoyle en C₁-C₆, un trihalogèno-méthyle, un alcoxy en C₁-C₆, un alcane en C₁-C₆ sulfonyloxy, un aminocarbonyle, un aminocarbonyle contenant un ou deux radicaux alcoyle en C₁-C₆, un alcoyle en C₁-C₆-carbonyle, un amino, un dialcoyle en C₁-C₆ amino, un (alcoyle en C₁-C₆)₃ N, un alcanoyle en C₁-C₆ amino, un alcoyle en C₁-C₆ - alcanoyle en C₁-C₆ amino, un alcane en C₁-C₆ sulfonylamino, un alcoyle en C₁-C₆, alcane en C₁-C₆ sulfonylamino, un aminosulfonyle, un aminosulfonyle contenant un ou deux radicaux alcoyle en C₁-C₆, un alcoxy en C₁-C₆ sulfonyle(-SO₂-O-C₁-C₆ alcoyle), un sulfo ou alcolane en C₁-C₆ sulfonyle ou deux de ces restes peuvent également être le groupe méthylène dioxy et X est, respectivement OH, CH₃O, C₆H₅O, F, Cl, Br, I, C₂H₅O, C₃H₇O, t-C₄H₉O, AcO, SCN, CN et 0/2 (µ-oxo).

3. Procédé d'obtention des complexes oxo du tungstène (V)-porphyrine WO(P)X selon la revendication 1 ou 2 caractérisé en ce que l'on condense une mole de 5,10,15,20-tétraphénylporphyrine ou de 5,10,15,20-tétra(4-pyridyl)-porphyrine avec au moins une mole de pentahalogénure de tungstène dans un solvant à haut point d'ébullition qui dissout tous les réactants, à des températures allant de 160 à 250°C, on élimine le solvant, on fractionne le résidu après reprise dans un solvant organique, par chromatographie sur colonne, puis on condense le complexe qui se trouve dans la fraction principale encore hétérogène en ce qui concerne la composition de l'anion X⁻ du complexe W(P)X, que l'on traite soit respectivement avec une quantité équimolaire d'un composé de métal alcalin formé éventuellement in situ, qui contient l'anion X-désiré en liaison avec le métal alcalin à 0-100°C dans un mélange de solvants dans lequel aussi bien WO(P)X que le composé de métal alcalin se dissout et ON amène ensuite le complexe oxo du tungstène (V)porphyrine WO(P)X qui porte l'anion désiré X⁻ à la cristallisation par concentration, soit cependant respectivement on traite à 20-180°C, avec un excès de l'acide de Brönstedt HX correspondant à l'anion désiré X⁻ dans un mélange de solvants qui dissout aussi bien le complexe oxohalogèno du tungstène WO(P)X(X=F,Cl,Br,I) que l'acide de Brönstedt HX et à partir de là on amène à la cristallisation le complexe WO(P)X qui porte l'anion désiré X⁻ par concentration.

4. Procédé selon la revendication 3, caractérisé en ce que le solvant qui dissout tous les réactants est le benzonitrile ou le trichlorobenzène, en ce que les réactants sont mis à réagir dans ce solvant à des températures allant de 180 à 220°C et en ce que le solvant est chassé sous vide.

5. Procédé d'obtention des complexes oxo du tungstène (V) porphyrine WO(P)X selon la revendication 1 ou la revendication 2, caractérisé en ce que l'on verse une mole du ligand de porphyrine correspondant dans une solution bouillante d'au moins une mole de tétrachlorure du tungstène dans le benzonitrile ou le trichlorobenzène, tandis que le tétrachlorure de tungstène est préparé au préalable "in situ" par réaction de 1 mol de tungstène hexacarbonyle avec 2 mol d'hexachlorure de tungstène dans ce solvant et ne doit pas être isolé et on fait réagir cette solution à des températures de 160 à 250°C, on élimine le solvant sous vide, on fractionne le résidu après reprise dans un solvant organique, par chromatographie sur colonne, on condense le complexe encore hétérogène WO(P)X en ce qui concerne la composition de l'anion X⁻ et qui se trouve dans la fraction principale, on traite soit respectivement avec une quantité équimolaire d'un composé de métal alcalin obtenu, le cas échéant, "in situ" qui contient l'anion désiré X⁻ en liaison avec le métal alcalin, à 0-100°C, dans un mélange de solvants qui dissout aussi bien WO(P)X que le composé de métal alcalin et à partir de cela on amène le complexe WO(P)X qui porte l'anion désiré X⁻ à la cristallisation par concentration, ou on traite respectivement avec un excès de l'acide de Brönstedt HX correspondant à l'anion désiré X⁻ à 20-180°C, dans des mélanges de solvants qui dissolvent aussi bien le complexe halogèno-oxo du tungstène (V) que l'acide de Brönstedt et on amène ensuite le complexe WO(P)X qui porte l'anion désiré X⁻ à la cristallisation par concentration.

6. Procédé selon la revendication 5, caractérisé en ce que l'on fait réagir le ligand de porphyrine respectif avec le tétrachlorure de tungstène dans le solvant benzonitrile ou trichlorobenzène à des températures allant de 180 à 220°C et que l'on élimine le solvant sous vide.
